# EUROPEAN PATENT SPECIFICATION

(11) **EP 1 268 746 B1**
(45) Date of publication and mention of the grant of the patent: **18.11.2009**
(21) Application number: 01921047.5
(22) Date of filing: 03.04.2001
(51) Int. Cl.: C12N 5/06, C12N 1/38, C07K 14/52, C07K 14/42, C07K 16/00, A61K 35/14, A61K 35/00, A61K 31/00

(54) **PRODUCTION OF TcR GAMMA DELTA T CELLS**
HERSTELLUNG VON TCR GAMMA DELTA T-ZELLEN
PRODUCTION DE LYMPHOCYTES T GAMMA DELTA

(30) Priority: 03.04.2000 US 194033 P
(43) Date of publication of application: 02.01.2003
(73) Proprietor: Therapure Biopharma Inc., Mississauga ON L5N 8H9 (CA)
(72) Inventor: BELL, David, N., Oakville, Ontario L6H 4A7 (CA); SKEA, Danna, L., Mississauga, Ontario L5M 5W9 (CA)
(74) Representative: Harding, Charles Thomas
(86) International application number: PCT/CA2001/000444
(87) International publication number: WO 2001/075072

(56) References cited:
- WO-A-00/26347
- WO-A-98/33891
- WO-A-99/46365
- HEDGE R ET AL: "Large ex vivo expansion of cytotoxic TcRgammadelta+ T lymphocytes." BLOOD, vol. 92, no. 10 SUPPL. 1 PART 1-2, 15 November 1998 (1998-11-15), page 59B XP001033536 40th Annual Meeting of the American Society of Hematology;Miami Beach, Florida, USA; December 4-8, 1998 ISSN: 0006-4971
- YAMAGUCHI TOMOHIRO ET AL: "A simple method for the propagation and purification of gamma-delta-T cells from the peripheral blood of glioblastoma patients using solid-phase anti-CD3 antibody and soluble IL-2." JOURNAL OF IMMUNOLOGICAL METHODS, vol. 205, no. 1, 1997, pages 19-28, XP004082106 ISSN: 0022-1759 cited in the application
- YAMAGUCHI TOMOHIRO ET AL: "Interleukin-15 effectively potentiates the in vitro tumor-specific activity and proliferation of peripheral blood gammadeltaT cells isolated from glioblastoma patients." CANCER IMMUNOLOGY IMMUNOTHERAPY, vol. 47, no. 2, October 1998 (1998-10), pages 97-103, XP001033539 ISSN: 0340-7004 cited in the application
- BARCENA A ET AL: "A ROLE FOR INTERLEUKIN 4 IN THE DIFFERENTIATION OF MATURE T CELL RECEPTOR GAMM/DELTA+ CELLS FROM HUMAN INTRATHYMIC T CELL PRECURSORS" JOURNAL OF EXPERIMENTAL MEDICINE, TOKYO, JP, vol. 172, no. 2, 1 August 1990 (1990-08-01), pages 439-446, XP000876697 ISSN: 0022-1007
- BOISMENU RICHARD ET AL: "An innate view of gamma-delta T cells." CURRENT OPINION IN IMMUNOLOGY, vol. 9, no. 1, 1997, pages 57-63, XP001030660 ISSN: 0952-7915
- WALLACE MARIANNE ET AL: "Gamma/delta T lymphocytes in viral infections." JOURNAL OF LEUKOCYTE BIOLOGY, vol. 58, no. 3, 1995, pages 277-283, XP001033538 ISSN: 0741-5400
- SALERNO ALFREDO ET AL: "Role gammadelta T lymphocytes in immune response in humans and mice." CRITICAL REVIEWS IN IMMUNOLOGY, vol. 18, no. 4, 1998, pages 327-357, XP001033531 ISSN: 1040-8401
- SKEA DANNA L ET AL: "Ex vivo expanded autologous TcRgammadelta+ T cells for the treatment of minimal residual disease following autotransplantation in chronic myelogenous leukemia." BLOOD, vol. 96, no. 11 Part 2, 16 November 2000 (2000-11-16), page 258B XP001030631 42nd Annual Meeting of the American Society of Hematology;San Francisco, California, USA; December 01-05, 2000 ISSN: 0006-4971

## Description

The present invention relates to novel and improved methods for the *ex vivo* expansion of TcRγδ⁺ T cells.

TcRγδ⁺ T cells are a small subset of circulating T lymphocytes that are distinct from conventional TcRαβ⁺ T cells which recognize, with fine specificity, foreign peptide antigens in the context of classical class I or class II major histocompatibility complex (MHC) restriction elements. By contrast, TcRγδ⁺ T cells are able to recognize both peptide and non-peptide antigens which may be derived from either foreign microorganisms or endogenous cellular products induced by stress such as viral infection or transformation. Moreover, unlike antigen recognition by TcRαβ⁺ T cells, antigen recognition by TcRγδ⁺ T cells is not MHC-restricted.

The T cell receptors of TcRαβ⁺ and TcRγδ⁺ T cells are distinguished by the different genetic elements that encode them. The majority of TcRγδ⁺ T cells are classified into two main subsets, Vδ1⁺ and Vδ2⁺, based on the genes that encode their δ chain. The major subset of TcRγδ⁺ T cells in human peripheral blood expresses Vδ2 in combination with Vγ9, while most of the remainder express Vδ1 in combination with Vγ2, Vγ3, Vγ4, Vγ5 or Vγ8 (Salerno, A. and Dieli, F., 1998).

Since TcRγδ⁺ T cells lack the fine specificity characteristics of TcRαβ⁺ T cells, it has been proposed that they represent a more primitive immune mechanism that provides a first-line surveillance function against infection and tumours (Boismenu, R. et al., 1997). Several studies have documented the response of TcRγδ⁺ T cells to various viruses, bacteria and parasites (Bukowski, J.F. et al., 1994; Wallace, M. et al., 1995; Lang, F. et al., 1995; Elloso, M.M. et al., 1996) as well as their ability to mediate lysis of tumour cells of various origins (Zocchi, M.R. et al., 1990; Kitayama, J. et al., 1993; Choudhary, A. et al., 1995). Hematopoietic tumours may be particularly susceptible to the lytic effects of TcRγδ⁺ T cells, since transgenic mice expressing the Vγ1.1 transgene display spontaneous resistance to injected T cell leukemias, and TcRγδ⁺ T cell hybridomas derived from these mice preferentially respond to hematopoietic malignant cells over non-hematopoietic tumour cells (Penninger, J. et al., 1995). Moreover, human TcRγδ⁺ T cell clones derived from patient peripheral blood and bone marrow have been shown to lyse autologous leukemic cells in acute lymphoblastic leukemia and acute myeloid leukemia, respectively (Bensussan, A. et al., 1989; Jahn, B. et al., 1995). Furthermore, improved disease-free survival in leukemia patients after allogeneic bone marrow transplantation has been shown to be associated with an increase in the number and percentage of TcRγδ⁺ T cells in peripheral blood (Lamb, L.S. et al., 1996). Collectively, these results suggest that TcRγδ⁺ T cells may have therapeutic potential in the treatment of cancer and infectious diseases.

Many of the published methods describing the *ex vivo* expansion of TcRγδ⁺ T cells require the presence of antigen. Virus-infected or transformed cells or cell lines, bacteria and parasites have been shown to stimulate TcRγδ⁺ T cell expansion *ex vivo,* as have established tumour cell lines. For example, herpes simplex virus (HSV)-infected cells were used to stimulate the expansion of Vδ2⁺ cells (Bukowski, J.F. et al., 1994), while Epstein-Barr virus (EBV)-transformed B-lymphoblastoid cell lines were used to stimulate the expansion of Vδ1⁺ cells (Orsini, D.L.M. et al., 1993). Extracts of *Mycobacterium tuberculosis* and blood-stage *Plasmodium falciparum* malarial antigens have been shown to stimulate proliferation of TcRγδ⁺ T cells (Constant, P. et al., 1994; Elloso, M.M. et al., 1996). Daudi, an immortalized human Burkitt's lymphoma cell line, can also stimulate the proliferation of TcRγδ⁺ T cells (Kaur, I. et al., 1993). In addition, well-characterized, non-peptidyl antigens of the prenyl phosphate family, for example, isopentenyl pyrophosphate, have been shown to stimulate the *ex vivo* expansion of TcRγδ⁺ T cells (Garcia, V.E. et al., 1998). In some of these systems, the antigen-stimulated cultures of TcRγδ⁺ T cells were supplemented with cytokines.

TcRγδ⁺ T cells have also been expanded *ex vivo* from populations of tumour infiltrating lymphocytes (TIL) by culture with IL-2 (Zocchi, M.R. et al., 1990) or IL-2 in combination with immobilized anti-CD3 antibody (Kitayama, J. et al., 1993) or anti-TcRγδ⁺ antibody (Yu,S. et al., 1999). In these systems, selective stimulation of the TcRγδ⁺ T cells by the tumour antigens is presumed to have occurred *in vivo* prior to isolation of T cells from the cancerous tissue.

In another system, TcRγδ⁺ T cells were expanded from the peripheral blood of glioblastoma patients using a solid-phase, immobilized anti-CD3 antibody in combination with IL-2 followed by culture in IL-2 alone (Yamaguchi, T., et al, 1997). These authors reported that the subsequently purified TcRγδ⁺ T cells did not proliferate for more than one week in the presence of IL-2 alone and therefore, they concluded, that this method would be applicable only to short term studies. They further showed that the method resulted in the expansion and enrichment of both TcRγδ⁺ and TcRαβ⁺ T cells, achieving TcRγδ⁺ T cell purities on the order of 28%. In a subsequent report, the same authors demonstrated that this method selectively expanded the Vδ2⁺ subset (Suzuki, Y., et al, 1999). In another report, this group showed that TcRγδ⁺ T cells which were expanded in culture using anti-CD3 and IL-2 followed by IL-2 alone and which were subsequently purified from the expanded population proliferated better in a ³H-thymidine incorporation assay when IL-15 was added (Yamaguchi, et al, 1998).

Thus, there are limitations to cell proliferation and/or requirements for antigen stimulation in the existing methods for *ex vivo* culture and expansion of TcRγδ⁺ T cells. In view of the foregoing, there is a need in the art for a method to selectively culture large amounts of essentially pure TcRγδ⁺ cells *in vitro*.

The present invention provides novel methods for expanding TcRγδ⁺ T cells in culture in the absence of exogenous antigen. In particular, the inventors have shown that TcRγδ⁺ T cells can be expanded by culturing the cells in a first media containing a T cell mitogen, interleukin 2 and interleukin 7 and then sub-culturing the cells in a second culture medium containing interleukin 2 and interleukin 7 in the absence of the mitogen. Accordingly, the present invention provides a method for expanding TcRγδ⁺ T cells in a starting sample comprising:
(1) culturing cells in the starting sample in a first culture medium comprising a T cell mitogen, interleukin-2 and interleukin-7; and
(2) culturing the cells obtained in step (1) in a second culture medium comprising interleukin-2 and interleukin-7 to expand TcRγδ⁺ T cells.

The term "a growth factor having interleukin-2-like activity" means any compound that has the same activity as IL-2 with respect to its ability to expand TcRγδ⁺ T cells in culture and includes, but is not limited to, IL-2 and IL-2 mimetics, or any functional equivalent of IL-2.

The term "a growth factor having interleukin-7-like activity" means any compound that has the same activity as IL-7 with respect to its ability to expand TcRγδ⁺ T cells in culture and includes, but is not limited to, IL-7, IL-7 mimetics, or any functional equivalent of IL-7.

The methods of the invention result in expanded cell populations of TcRγδ⁺ T cells. By "expanded" it is meant that the number of the desired or target cell type (i.e., TcRγδ⁺ T cells) in the final preparation is higher than the number in the initial or starting cell population.

In a preferred embodiment, prior to culturing the cells in the first culture medium, the cells are depleted of non- TcRγδ⁺ cells. The first and second culture media may also contain other growth factors (in addition to IL-2 and IL-7) that can enhance the expansion of the TcRγδ⁺ T cells. Examples of such growth factors include IL-4 and IL-15.

The TcRγδ⁺ T cells obtained by the method of the invention can be used in a variety of experimental, therapeutic and commercial applications.

Other features and advantages of the present invention will become apparent from the following detailed description. It should be understood, however, that the detailed description and the specific examples while indicating preferred embodiments of the invention are given by way of illustration only, since various changes and modifications will become apparent to those skilled in the art from this detailed description.

The invention will now be described in relation to the drawings in which:
Figure 1 is a graph showing the total viable cells versus time under various culture conditions.
Figure 2 is a graph showing the total viable cells versus time under various culture conditions.
Figure 3 is a graph showing the total viable cells versus time under various culture conditions.
Figure 4 is a graph showing the total viable cells versus time under various culture conditions.
Figure 5 is a graph showing the total viable cells versus time under various culture conditions.

The present invention provides novel methods for selectively expanding TcRγδ⁺ T cells in culture. The methods can use either unfractionated starting samples or starting samples which have been enriched for T cells. Advantageously, the methods of the invention do not require the use of antigenic stimulation which is necessary in most other procedures.

Accordingly, the present invention provides a method for expanding TcRγδ⁺ T cells in a starting sample comprising:
(1) culturing cells in the starting sample in a first culture medium comprising a T cell mitogen and at least two growth factors; and
(2) culturing the cells obtained in step (1) in a second culture medium comprising at least two growth factors to expand TcRγδ⁺ T cells,
in which the two growth factors are any growth factors that can expand TcRγδ⁺ T cells in culture, i.e., IL-2 and IL-7.

We describe a method for expanding TcRγδ⁺ T cells in a starting sample comprising:
(1) culturing cells in the starting sample in a first culture medium comprising (a) a T cell mitogen, (b) a growth factor having interleukin-2-like activity and (c) a growth factor having interleukin-7-like activity; and
(2) culturing the cells obtained in step (1) in a second culture medium comprising (i) a growth factor having interleukin-2-like activity and (ii) a growth factor having interleukin-7-like activity to expand TcRγδ⁺ T cells.

The term "a growth factor having interleukin-2-like activity" means any compound that has the same activity as IL-2 with respect to its ability to expand TcRγδ⁺ T cells in culture and includes, but is not limited to, IL-2 and IL-2 mimetics, or any functional equivalent of IL-2.

The term "a growth factor having interleukin-7-like activity" means any compound that has the same activity as IL-7 with respect to its ability to expand TcRγδ⁺ T cells in culture and includes, but is not limited to, IL-7, IL-7 mimetics, or any functional equivalent of IL-7.

The starting sample can be any sample that contains TcRγδ⁺ T cells or precursors thereof including, but not limited to, blood, bone marrow, lymphoid tissue, epithelia, thymus, liver, spleen, cancerous tissues, lymph node tissue, infected tissue, fetal tissue and fractions or enriched portions thereof. The starting sample is preferably blood including peripheral blood or umbilical cord blood or fractions thereof, including buffy coat cells, mononuclear cells and low density mononuclear cells (LDMNC). The cells may be obtained from a starting sample of blood using techniques known in the art such as density gradient centrifugation. For example, whole blood may be layered onto an equal volume of Ficoll-Hypaque™ followed by centrifugation at 400xg for 30 minutes at room temperature. The interface material will contain the low density mononuclear cells which can be collected and washed in culture medium and centrifuged at 100xg for 10 minutes at room temperature. Prior to culturing for TcRγδ⁺ cells, the cells can be maintained in any suitable mammalian culture medium such as AIM-V™, RPMI 1640 or IMDM.

Prior to culturing the starting sample or fraction thereof (such as LDMNC) in the first culture medium, the sample or fraction thereof may be enriched for certain cell types and/or depleted for other cell types. In particular, the starting sample or fraction thereof may be enriched for T cells together with the depletion of TcRαβ⁺ T cells. The sample may be enriched or depleted of certain cell types using techniques known in the art. In one embodiment the cells of a particular phenotype may be depleted by culturing the starting sample or fraction thereof with an antibody cocktail containing antibodies specific for markers on the cells to be depleted. Preferably, the antibodies in the cocktail are tetrameric antibody complexes as described in United States Patent No. 4,868,109 to Lansdorp.

Once the cells in the starting sample have been fractionated and enriched, if desired, the cells are cultured in a first culture medium comprising a T cell mitogen and at least two growth factors, interleukin-2 and interleukin-7. Preferably, the T cell mitogen is present in an amount from about 0.01 to about 100 µg/ml; the interleukin-2 is present in an amount from about 0.1 to about 1000 ng/ml; the.interleukin-7 is present in an amount from about 0.1 to about 1000 ng/ml. More preferably, the T cell mitogen is present in an amount from about 0.1 to about 50 µg/ml; the interleukin-2 is present in an amount from about 1 to about 100 ng/ml; the interleukin-7 is present in an amount from about 1 to about 100 ng/ml. Most preferably, the medium comprises 1 µg/mL of a T cell mitogen; 10 ng/mL of interleukin-2 and 10 ng/mL of interleukin-7.

The cells are preferably cultured in the first culture medium for a period of time ranging from about 3 days to about 21 days. More preferably, from about 5 days to about 14 days.

The T cell mitogen can be any agent that can stimulate T cells including, but not limited to, lectins of plant and non-plant origin, antibodies that activate T cells, and other non-lectin/non-antibody mitogens. A preferred plant lectin is concanavalin A (ConA) although other plant lectins such as phytohemagglutinin (PHA) may be used. Preferred antibodies include an anti-CD3 antibody such as OKT3, an anti-CD2 antibody such as OKT11 or an anti-CD28 antibody. Within the context of the present invention, antibodies are understood to include monoclonal antibodies, polyclonal antibodies, antibody fragments (e.g., Fab, and F(ab¹)2), single chain antibodies, single chain variable fragments(ScFv) and recombinantly produced binding partners. Other mitogens include phorbol 12-myristate-13-acetate (TPA) and its related compounds, such as mezerein, or bacterial compounds such as Staphylococcal enterotoxin A (SEA) and Streptococcal protein A. The T cell mitogen may be soluble or immobilized and more than one T cell mitogen may be used in the method of the invention.

Following culture in the first culture medium, the cells are washed by centrifugation and sub-cultured in a second culture medium comprising at least two growth factors, including at least interleukin-2 and interleukin-7. If the cells are sub-cultured with IL-2 alone then proliferation continues for a few days but then quickly abates. Thus, for continued cell proliferation following the removal of the mitogen, both IL-2 and IL-7 are required in the second culture medium.

The sub-culture step (i.e. removal of the T cell mitogen) is important for the expansion of TcRγδ⁺ T cells by the method of the present invention particularly if the starting sample or LDMNC are not fractionated prior to culture in the first culture medium. If the LDMNC are fractionated then the subculture step may be optional. Conversely, if mitogen is left out of the first culture medium, little/no cell expansion occurs: The removal of mitogen by sub-culture has the further advantage of making the TcRγδ⁺ T cells better suited for therapeutic use, as the administration of residual mitogen to a patient is not desirable. The removal of the T cell mitogen in the subculturing step may not be required if the TcRγδ⁺ T cells are for experimental, diagnostic or other non-therapeutic uses.

Preferably, in the second culture medium, the two growth factors IL-2 and IL-7 are present in the same concentrations as listed above for the first culture medium.

The cells are preferably cultured in the second culture medium for a period of time ranging from about 3 days to about 21 days. More preferably, from about 9 days to about 13 days.

The first and second culture media may additionally include other ingredients that can assist in the growth and expansion of the TcRγδ⁺ T cells. Examples of other ingredients that may be added, include, but are not limited to, plasma or serum, additional growth factors including cytokines such as IL-4 and IL-5, tumour necrosis factors (TNFs) and interferons (TFNs), purified proteins such as albumin, a lipid source such as low density lipoprotein (LDL), vitamins, amino acids, steroids and any other supplements supporting or promoting growth and/or survival.

In a preferred embodiment, the first and second culture media additionally include other growth factors such as IL-4 or IL-15 or a mimetic or functional equivalent thereof. The inventors have found that the addition of a third growth factor to the first and second media enhances the expansion of the TcRγδ⁺ T cells as compared to the expansion obtained using two growth factors.

Accordingly, we further describe a method for expanding TcRγδ⁺ T cells in a starting sample comprising:
(1) culturing cells in the starting sample in a first culture medium comprising a T cell mitogen and at least three growth factors; and
(2) culturing the cells obtained in step (1) in a second culture medium comprising at least three growth factors to expand TcRγδ⁺ T cells.

In a preferred embodiment, the three growth factors are selected from combinations of IL-2, IL-4, IL-7; and IL-15. Examples of combinations include IL-2, IL-4 and IL-15; IL-2, IL-4 and IL-7 and IL-2, IL-7 and IL-15. The third growth factor can be present in an amount similar to the two growth factors as described above.

The inventors have also found that addition of a fourth growth factor to the first and second media enhances the expansion of the TcRγδ⁺ T cells as compared to the expansion obtained using two or three growth factors.

Accordingly, we further describe a method for expanding TcRγδ⁺ T cells in a starting sample comprising:
(1) culturing cells in the starting sample in a first culture medium comprising a T cell mitogen and at least four growth factors; and
(2) culturing the cells obtained in step (1) in a second culture medium comprising at least four growth factors to expand TcRγδ⁺ T cells.

In preferred embodiments, the four growth factors are IL-2, IL-4, IL-7 and IL-15.

Preferably, both the first and second culture media are supplemented with serum or plasma (P). The amount of P in the first and second culture media is preferably from about 1% to about 25%. More preferably, the amount of P in the first and second culture media is from about 2% to about 20%. Even more preferably, the amount of P in the first and second culture media is from about 2.5% to about 10%. Most preferably, the amount of P is the first and second culture media is 5%. The serum or plasma (P) can be obtained from any source including, but not limited to, human peripheral blood, umbilical cord blood, or blood derived from another mammalian species. The plasma may be from a single donor or may be pooled from several donors. If autologous TcRγδ⁺ T cells are to be used clinically, i.e. reinfused into the same patient from whom the original starting sample was obtained, then it is preferable to use autologous P as well (i.e. from the same patient) to avoid the introduction of extraneous products (e.g. viruses) into that patient. If the TcRγδ⁺ T cells are to be used allogeneically (i.e. infused into a person other than the one from whom the original starting sample was obtained) then it is preferable to use plasma from one or the other to minimize the introduction of extraneous products into the patient; at a minimum the plasma should be human-derived to avoid the administration of animal products to the patient.

The methods of the invention result in expanded cell populations of TcRγδ⁺ T cells. By "expanded" it is meant that the number of the desired or target cell type (i.e., TcRγδ⁺ T cells) in the final preparation is higher than the number in the initial or starting cell population.

The TcRγδ⁺ T cells obtained according to the methods of the invention can be separated from other cells that may be present in the final culture using techniques known in the art including fluorescence activated cell sorting, immunomagnetic separation, affinity column chromatography, density gradient centrifugation and cellular panning.

TcRγδ⁺ T cells obtained by the methods of the invention are also of use. Accordingly, we describe a cell preparation of TcRγδ⁺ T cells. Preferably, the TcRγδ⁺ T cells comprise greater than 60%, more preferably greater than 80% and most preferably greater than 90%, of the total cells in the enriched population.

The TcRγδ⁺ cells obtained by the method of the invention may be used in any and all applications. TcRγδ⁺ T cells are thought to be a first line of defense against infectious pathogens. In addition, TcRγδ⁺ T cells possess intrinsic cytolytic activity against transformed cells of various origins including B-cell lymphomas, sarcomas and carcinomas. As a result, the TcRγδ⁺ T cells obtained and cultured *ex vivo* according to the method of the invention, can be transfused into a patient for the treatment or prevention of infections, cancer or diseases resulting from immunosuppression. Advantageously, the TcRγδ⁺ T cells of the invention do not contain mitogen or fetal bovine serum making them useful for human therapeutic applications. Accordingly, we describe a method of modulating an immune response comprising administering an effective amount of TcRγδ⁺ T cells prepared according to a method of the invention to an animal in need thereof.

The term "effective amount" as used herein means an amount effective, at dosages and for periods of time necessary to achieve the desired results.

The term "animal" as used herein includes all members of the animal kingdom. Preferably, the animal is a mammal, more preferably a human.

We describe a method of treating an infection comprising administering an effective amount of TcRγδ⁺ T cells prepared according to the method of the invention to an animal in need thereof.

Examples of infections that may be treated include, but are not limited to, bacterial infections such as those caused by *Mycobacteria* (e.g. tuberculosis), viral infections such as those caused by herpes simplex virus (HSV), human immunodeficiency virus (HIV) or the hepatitis viruses, and parasitic infections such as those caused by *Plasmodium* (e.g. malaria)

We also describe a method for treating cancer comprising administering an effective amount of TcRγδ⁺ T cells prepared according to the method of the invention to an animal in need thereof.

Examples of cancer that may be treated include, but are not limited to, leukemias including chronic myelogenous leukemia, acute myelogenous leukemia, acute lymphoblastic leukemia, and T cell and B cell leukemias, lymphomas (Hodgkins and non-Hodgkins), lymphoproliferative disorders, plasmacytomas, histiocytomas, melanomas, adenomas, sarcomas, carcinomas of solid tissues, hypoxic tumours, squamous cell carcinomas, genitourinary cancers such as cervical and bladder cancer, hematopoietic cancers, head and neck cancers, and nervous system cancers.

We further describe a method of treating chronic myelogenous leukemia comprising administering an effective amount of TcRγδ⁺ T cells prepared according to the method of the invention to an animal in need thereof. In such an embodiment, the LDMNC can be obtained from a patient with chronic myelogenous leukemia (CML). After culturing and expanding for TcRγδ⁺ T cells, the expanded cells will not contain cancerous CML cells making them well suited for re-infusion back to the-patient.

We describe the use of the TcRγδ⁺ T cells obtained by the methods of the invention to modulate an immune response, to treat an infection or to treat cancer as described hereinabove. The invention further includes the use of the TcRγδ⁺ T cells obtained according to the methods of the invention to prepare a medicament or pharmaceutical composition to modulate an immune response, to treat an infection or to treat cancer as described hereinabove.

The TcRγδ⁺ T cells obtained according to the present invention can also be used in experimental models, for example, to further study and elucidate the function of the cells. Additionally, these cells may be used for studies directed towards the identification of the antigens/epitopes recognized by TcRγδ⁺ T cells and for the design and development of vaccines.

The obtained TcRγδ⁺ T cells, according to the invention may be immediately used in the above therapeutic, experimental or commercial applications or the cells may be cryopreserved for use at a later date.

The following non-limiting examples are illustrative of the present invention:

### EXAMPLES

### EXAMPLE 1

Low density mononuclear cells (LDMNC) were isolated from adult peripheral blood by density gradient centrifugation using Ficoll-Hypaque™ (density = 1.077 g/ml). A volume of 100 ml of whole blood was layered in 10 - 15 ml aliquots onto an equal volume of Ficoll-Hypaque™ in 50 ml conical tissue culture tubes which were then centrifuged at 400 x g for 30 minutes at room temperature. The interphase material containing the mononuclear cells was collected and the cells were washed twice in culture medium (AIM-V™ containing 20 units/ml of heparin and 50 µM 2-mercaptoethanol; HCBM-2) by centrifugation at 100 x g for 10 minutes at room temperature. The cells were diluted in HCBM-2 containing 10% autologous plasma and incubated in polystyrene tissue culture flasks overnight at 37°C and 5% CO₂. The next morning, the cells were washed twice by centrifugation and resuspended in phosphate buffered saline (PBS) containing 2% autologous plasma. A sample of the cell suspension was diluted 1:20 with 2% acetic acid and the total number of nucleated cells determined using a hemocytometer.

TcRγδ⁺ T cells were enriched from the LDMNC by negative selection using a cocktail of lineage specific antibodies and immunomagnetic affinity chromatography (StemSep™, Stem Cell Technologies, Vancouver, BC). A total of 8.2 x 10⁷ LDMNC were resuspended in 1.25 ml of PBS containing 2% autologous plasma and a cocktail of lineage specific, monoclonal antibodies was added. The cocktail contained antibodies specific for CD14 (monocytes), CD16 (NK cells), CD19 (B cells), CD33 (myeloid progenitor cells), CD56 (NK cells), glycophorin A (erythroid cells) and TcRαβ (TcRαβ⁺ T cells). These were bispecific antibodies with one antigenic specificity for the lineage specific marker and the other antigenic specificity for dextran. The LDMNC were incubated with the bispecific antibodies on ice for 30 minutes following which iron dextran colloid was added and the incubation was continued for a further 30 minutes. The suspension was then subjected to immunomagnetic chromatography, a procedure which removed those cells which had been cross-linked by the bispecific antibodies to the iron dextran particles. Thus, the cells recovered were an enriched population of TcRγδ⁺ T cells that had not been bound by antibody or iron dextran. The yield of enriched TcRγδ⁺ T cells obtained was 2.65 x 10⁵ cells.

The enriched TcRγδ⁺ T cells were seeded into tissue culture under various conditions (described below) at a density of 1 x 10⁵ cells /ml in HCBM-2 containing 5% autologous plasma (P) and the cultures were incubated at 37°C and 5% CO₂. At various time points, cell expansion was monitored by counting the cells in a small sample of each culture using a hemocytometer. The samples were diluted with an equal volume of 0.4% trypan blue prior to counting, so that the viable cells (unstained) could be distinguished from the non-viable cells (stained blue).

Initially, the cells were cultured under two different conditions (Condition 1 and Condition 6) and then the cells in Condition 1 were sub-cultured under five different conditions (Conditions 1-5). Initially, in Condition 1, a combination of mitogen and cytokines was added to 1.3 x 10⁵ enriched TcRγδ⁺ T cells as follows: 1 ug/ml of concanavalin A (ConA), 10 ng/ml of IL-2 and 10 ng/ml of IL-4. The cells were cultured in this condition for 7 days, after which they had expanded approximately 3.5-fold to a total of 4.5 x 10⁵ viable cells. At this point, fresh culture medium containing 5% autologous plasma was added to the culture to dilute the cells to a density of 1 x 10⁵ cells/ml (total volume = 4.5 ml) and 1 ug/ml of concanavalin A, 10 ng/ml of IL-2 and 10 ng/ml of IL-4 were added. The culture was continued for a further 2 days, after which the cells had expanded to a total of 2.0 x 10⁶ viable cells. At this point, the cells were sub-cultured as follows: the cells were pelleted by centrifugation at 100 x g for 10 minutes at room temperature, washed once with HCBM-2 and resuspended at 1.0 x 10⁵ cells/ml in HCBM-2 containing 2% autologous plasma. The resuspended cells were divided into 5 equal portions of 4.0 x 10⁵ cells each and were cultured under the following conditions:
1. 1 ug/ml ConA + 10 ng/ml IL-2 + 10 ng/ml IL-4 + 2% P
2. 10 ng/ml IL-2 + 10 ng/ml IL-4 + 2% P
3. 10 ng/ml IL-2 + 2% P
4. 10 ng/ml IL-4 + 2% P
5. 2% P

At day 12, the cells in each culture were counted and fresh culture medium containing 2% autologous plasma was added to each to dilute the cells to 1 x 10⁵ cells/ml. Fresh mitogen and cytokines were added to maintain the conditions given above. The cultures were continued for another 4 days (to day 16), at which point the cells in each condition were counted and analyzed by flow cytometry to determine the percentage of TcRγδ⁺ T cells.

In a sixth culture condition, the enriched TcRγδ⁺ T cells were seeded into culture as described above except in this condition the cells were never stimulated with mitogen and were maintained throughout in the presence of 10 ng/ml of IL-2 and 10 ng/ml of IL-4 in HCBM-2 with autologous plasma.

### Summary of Conditions:

Culture Condition → Sub-culture Condition

1. Con A + IL-2 + IL-4 + P → Con A + IL-2 + IL-4 + P
2. Con A + IL-2 + IL-4 + P → IL-2 + IL-4 + P
3. Con A + IL-2 + IL-4 + P → IL-2 + P
4. Con A + IL-2 + IL-4 + P → IL-4 + P
5. Con A + IL-2 + IL-4 + P → P
6. IL-2 + IL-4 + P

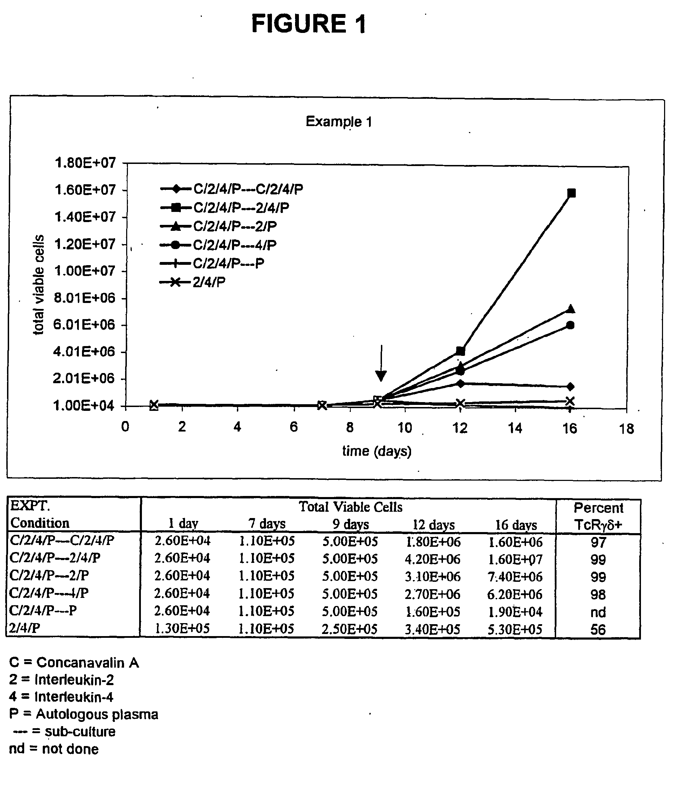

The results of this experiment are shown in Figure 1 in which the total number of viable cells in each culture condition is plotted as a function of time. The arrow in the figure indicates the time point at which the cells were sub-cultured. The numerical cell counts are given in the table accompanying the graph, together with the percentage of TcRγδ⁺ T cells in the expanded populations at the end of the culture period.

These data demonstrate the expansion of TcRγδ⁺ T cells by the method of the present invention, i.e.

Mitogen (e.g. Con A) + IL-2 + IL-4 → IL-2 + IL-4

The data also demonstrate that the expansion of TcRγδ⁺ T cells is greater when IL-4 is used than when it is not, i.e. the total expansion was higher in condition 2 compared to condition 3.

The data further demonstrate that the mitogen is necessary at the beginning of the culture for significant expansion of TcRγδ⁺ T cells to occur, i.e. the total expansion was much higher in condition 2 compared to condition 6.

The data further demonstrate that the sub-culture step (i.e. removal of the mitogen from the culture) results in better expansion of TcRγδ⁺ T cells, i.e. the total expansion was higher in condition 2 compared to condition 1.

The data also demonstrate that very pure TcRγδ⁺ T cells (99%) are obtained by the method of the present invention without the need for further purification of the expanded population of cells.

### EXAMPLE 2

TcRγδ⁺ T cells were enriched from adult peripheral blood LDMNC as described in Example 1. The enriched TcRγδ⁺ T cells were seeded into tissue culture and expanded in a manner similar to that described in Example 1, condition 2. Specifically, cultures were initiated with (1) 1 ug/ml of concanavalin A and 10 ng/ml of IL-2, or (2) 1 ug/ml of concanavalin A and 10 ng/ml of IL-2 and 10 ng/ml of IL-4, or (3) 1 ug/ml of concanavalin A and 10 ng/ml of IL-2 and 10 ng/ml of IL-7. At day 10, the cells in each condition were sub-cultured, i.e. the mitogen was removed, and the cells were further expanded in (1) IL-2, or (2) IL-2 and IL-4, or (3) IL-2 and IL-7. At day 17, the cultures were terminated, final cell counts were determined and TcRγδ⁺ T cell purity was assessed by flow cytometry.

### Summary of Conditions:

Culture Condition → Sub-culture Condition

1. Con A + IL-2 + P → IL-2 + P
2. Con A + IL-2 + IL-4 + P → IL-2 + IL-4 + P
3. Con A + IL-2 + IL-7 + P → IL-2 + IL-7 + P

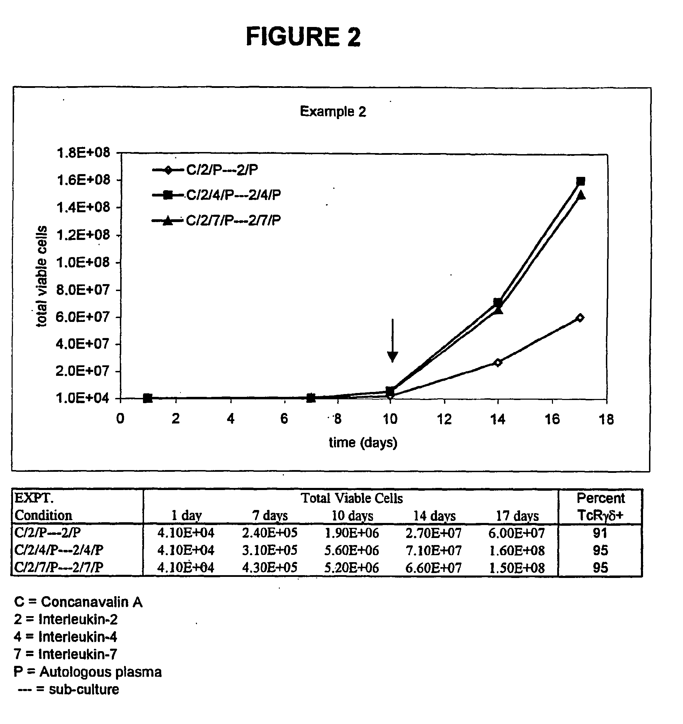

The results of this experiment are shown in Figure 2 in which the total number of viable cells in each culture condition is plotted as a function of time. The arrow in the figure indicates the time point at which the cells were sub-cultured (i.e. the mitogen was removed). The numerical cell counts are given in the table accompanying the graph, together with the percentage of TcRγδ⁺ T cells in the expanded populations at the end of the culture period.

These data demonstrate the expansion of TcRγδ⁺ T cells by the method of the present invention, i.e.

Mitogen (e.g. Con A) + IL-2 + IL-4 or IL-7 → IL-2 + IL-4 or IL-7

The data further demonstrate that the expansion of TcRγδ⁺ T cells is greater when IL-4 or IL-7 is used than when it is not, i.e. the total expansion was higher in conditions 2 and 3 compared to condition 1.

The data also demonstrate that very pure TcRγδ⁺ T cells (>95%) are obtained by the method of the present invention without further purification of the expanded population of cells.

### EXAMPLE 3

TcRγδ⁺ T cells were enriched from adult peripheral blood LDMNC as described in Example 1. The enriched TcRγδ⁺ T cells were seeded into tissue culture and expanded in a manner similar to that described in Example 1, condition 2, except that the mitogen used was an immobilized monoclonal antibody instead of concanavalin A. The monoclonal anti-CD3 antibody OKT3 was coated onto the wells of a tissue culture plate at a concentration of 10 ug/ml in PBS overnight at 4°C. The wells were emptied and washed five times with PBS prior to use. As a negative control, an isotype-matched immunoglobulin (IgG2a) of irrelevant antigenic specificity was similarly used. Cultures were initiated with (1) immobilized OKT3 and 10 ng/ml of IL-2, (2) immobilized OKT3 and 10 ng/ml of IL-2 and 10 ng/ml of IL-4, or (3) immobilized IgG2a and 10 ng/ml of IL-2 and 10 ng/ml of IL-4. At day 8, the cells in each condition were sub-cultured, i.e. the mitogen was removed. This was accomplished by resuspending the cells, removing the cell suspension from the coated wells, pelleting and washing the cells by centrifugation, and replacing the cells in uncoated tissue culture wells. The cultures were further expanded in (1) IL-2, (2) IL-2 and IL-4, or (3) IL-2 and IL-4. At day 20, the cultures were terminated, final cell counts were determined and TcRγδ⁺ T cell purity was assessed by flow cytometry.

### Summary of Conditions:

Culture Condition → Sub-culture Condition

1. OKT3 + IL-2 + P → IL-2 + P
2. OKT3 + IL-2 + IL-4 + P → IL-2 + IL-4 + P
3. IgG2a + IL-2 + IL-4 + P → IL-2 + IL-4 + P

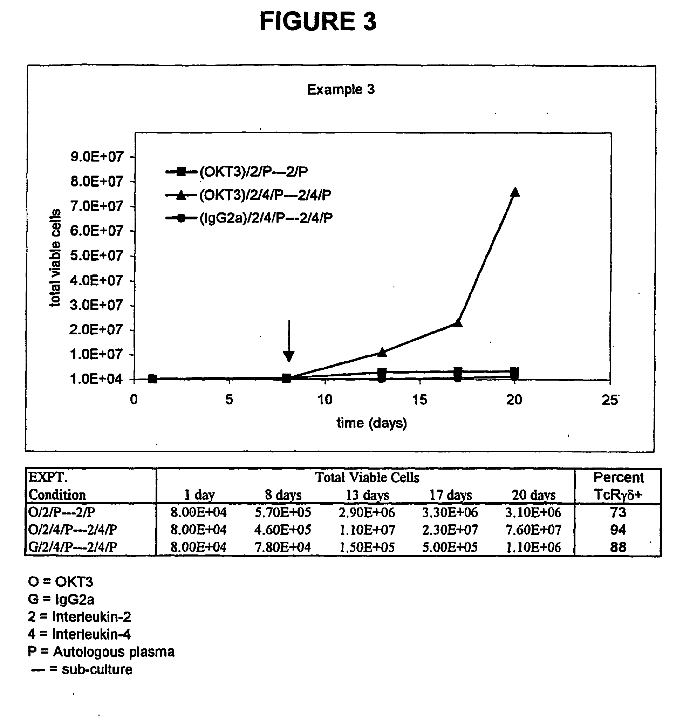

The results of this experiment are shown in Figure 3 in which the total number of viable cells in each culture condition is plotted as a function of time. The arrow in the figure indicates the time point at which the cells were sub-cultured. The numerical cell counts are given in the table accompanying the graph, together with the percentage of TcRγδ⁺ T cells in the expanded populations at the end of the culture period.

These data demonstrate the expansion of TcRγδ⁺ T cells by the method of the present invention, i.e.

Mitogen (e.g. OKT3) + IL-2 + IL-4 → IL-2 + IL-4

The data demonstrate that the expansion of TcRγδ⁺ T cells is greater when IL-4 is used than when it is not, i.e. the total expansion was higher in condition 2 compared to condition 1.

The data demonstrate that the mitogen can be a monoclonal antibody, and that the specificity of the monoclonal antibody is important since the total expansion was much higher in condition 2 compared to condition 3.

The data also demonstrate that very pure TcRγδ⁺ T cells (94%) are obtained by the method of the present invention without further purification of the expanded population of cells. Moreover, the purity of the TcRγδ⁺ T cells is higher when IL-4 is used (94%) than when it is not (73%).

### EXAMPLE 4

TcRγδ⁺ T cells were enriched from adult peripheral blood LDMNC as described in Example 1. The enriched TcRγδ⁺ T cells were seeded into tissue culture and expanded in a manner similar to that described in Example 3. Cultures were initiated with (1) immobilized OKT3 and 10 ng/ml of IL-2, (2) immobilized OKT3 and 10 ng/ml of IL-2 and 10 ng/ml of IL-4, or (3) immobilized OKT3 and 10 ng/ml of IL-2 and 10 ng/ml of IL-7. At day 6, the cells in each condition were sub-cultured, i.e. the mitogen was removed. The cultures were further expanded in (1) IL-2, (2) IL-2 and IL-4, or (3) IL-2 and IL-7. At day 21, the cultures were terminated, and final cell counts were determined.

### Summary of Conditions:

Culture Condition → Sub-culture Condition

1. OKT3 + IL-2 + P → IL-2 + P
2. OKT3 + IL-2 + IL-4 + P → IL-2 + IL-4 + P
3. OKT3 + IL-2 + IL-7 + P → IL-2 + IL-7 + P

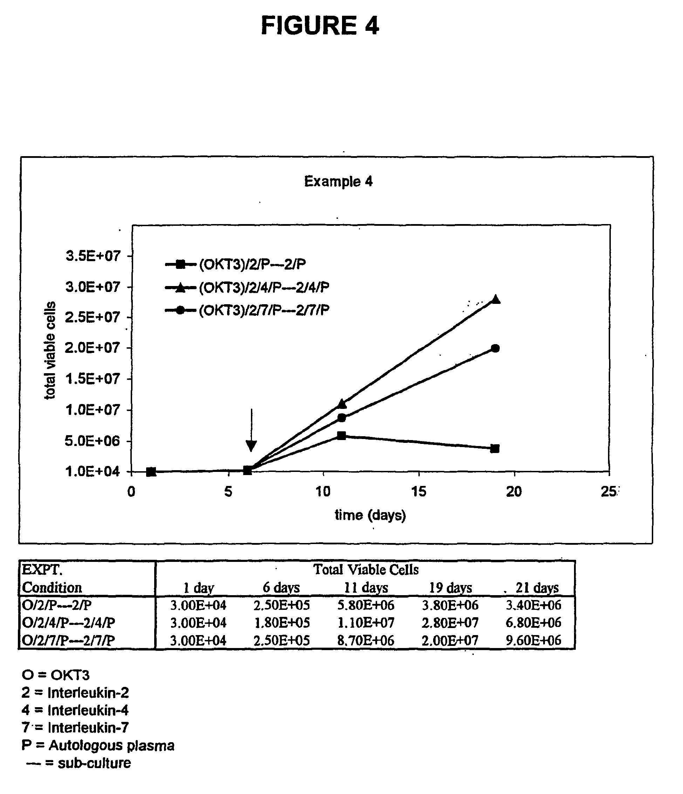

The results of this experiment are shown in Figure 4 in which the total number of viable cells in each culture condition is plotted as a function of time. The arrow indicates the time point at which the cells were sub-cultured. The numerical cell counts are given in the table accompanying the graph, together with the percentage of TcRγδ⁺ T cells in the expanded populations at the end of the culture period.

These data demonstrate the expansion of TcRγδ⁺ T cells by the method of the present invention, i.e.

Mitogen (e.g. OKT3) + IL-2 + IL-4 or IL-7 → IL-2 + IL-4 or IL-7

The data further demonstrate that the expansion of TcRγδ⁺ T cells is greater when IL-4 or IL-7 is used than when it is not, i.e. the total expansion was higher in conditions 2 and 3 compared to condition 1.

### EXAMPLE 5

TcRγδ⁺ T cells were enriched from adult peripheral blood LDMNC as described in Example 1. The enriched TcRγδ⁺ T cells were seeded into tissue culture and expanded in a manner similar to that described in Example 2. Specifically, cultures were initiated with 1 ug/ml of concanavalin A and a combination of cytokines (see below), each of which was used at 10 ug/ml. At day 8, the cells in each condition were sub-cultured, i.e. the mitogen was removed, and the cells were further expanded using the respective combination of cytokines alone.

### Summary of Conditions:

Culture Condition → Sub-culture Condition

1. Con A + IL-2 + P → IL-2 + P
2. Con A + IL-2 + IL-4 +P → IL-2 + IL-4 + P
3. Con A + IL-2 + IL-4 + IL-7 + P → IL-2 + IL-4 + IL-7 + P
4. Con A + IL-2 + IL-4 + IL-15 + P → IL-2 + IL-4 + IL-15 + P
5. Con A + IL-2 + IL-7 + IL-15 + P → IL-2 + IL-7 + IL-15 + P
6. Con A + IL-2 + IL-4 + IL-7 + IL-15 + P → IL-2 + IL-4 + IL-7 + IL-15 + P

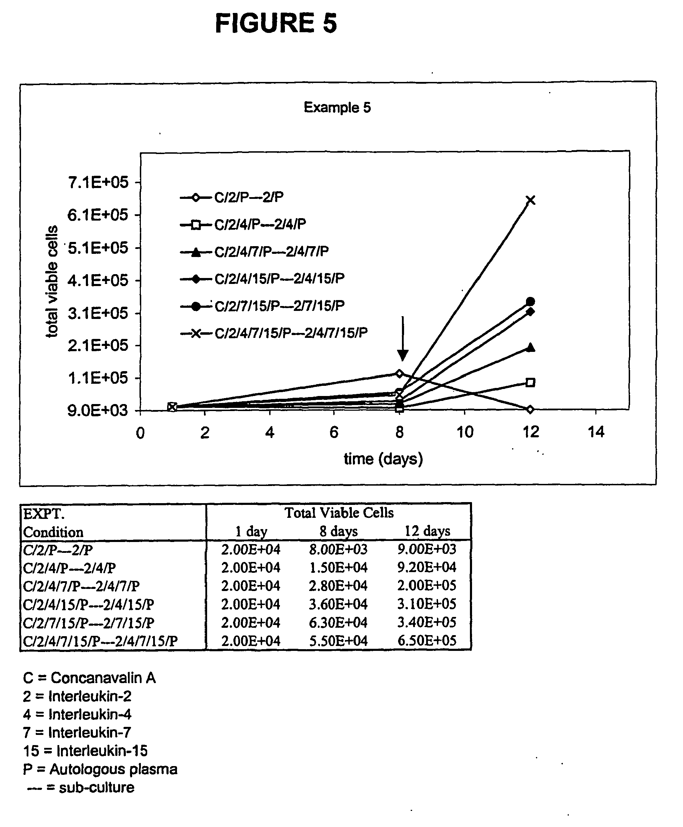

The results of this experiment are shown in Figure 5 in which the total number of viable cells in each culture condition is plotted as a function of time. The arrow in the figure indicates the time point at which the cells were sub-cultured (i.e. the mitogen was removed). The numerical cell counts are given in the table accompanying the graph.

These data demonstrate that the addition of a third growth factor enhances the expansion of the TcRγδ⁺ T cells as compared to the expansion obtained using one or two growth factors, i.e. the expansion was higher in conditions 3,4 and 5, compared to conditions 1 and 2.

These data further demonstrate that the addition of a fourth growth factor enhances the expansion of the TcRγδ⁺ T cells as compared to the expansion obtained using one or two or three growth factors, i.e. the expansion was higher in condition 6 compared to conditions 1-5.

### FULL CITATIONS FOR REFERENCES REFERRED TO IN THE SPECIFICATION

Bensussan, A., Lagabrielle, J.F., Castaigne, S., Boisson, N., Miclea, J.M., Benbunan, M., Degos, L. Human CD3 gamma delta + activated lymphocytes exhibit killer activity in vitro against autologous leukemia cells. Nouv Rev Fr Hematol 31:129, 1989.
Boismenu, R., Havran, W.L. An innate view of γδ T cells. Curr Op Immunol 9:57,1997.
Bukowski, J.F., Morita, C.T., Brenner, M.B. Recognition and destruction of virus-infected cells by human γδCTL. J. Immunol 153:5133, 1994.
Choudhary, A., Davodeau, F., Moreau, A., Peyrat, M.A., Bonneville, M., Jotereau, F. Selective lysis of autologous tumor cells by recurrent γδ tumor-infiltrating lymphocytes from renal carcinoma. J Immunol 154:3932, 1995.
Constant, P., Davodeau, F., Peyrat, M., Poquet, Y., Puzo, G., Bonneville, M. and Fournie, J. Stimulation of human γδ T cells by nonpeptidic mycobacterial ligands. Science 264:267,1994.
Elloso, M.M., Van der Heyde, H.C., Troutt, A., Manning, D.D. and Weidanz, W.P. Human gamma delta T cell subset-proliferative response to malarial antigen in vitro depends on CD4+ T cells or cytokines that signal through components of the IL-2R. J. Immunol. 157:2096,1996.
Garcia, V.E., Jullien, D., Song, M., Uyemura, K., Shuai, K, Morita, C.T. and Modlin, R.L. IL-15 enhances the response of human gamma delta T cells to non-peptide microbial antigens. J. Immunol. 160:4322,1998.
Jahn, B., Bergmann, L., Weidmann, E., Brieger, J., Fenchel, K., Schwulera, U., Hoelzer, D., Mitrou, P.S. Bone marrow-derived T-cell clones obtained from untreated acute myeolocytic leukemia exhibit blast directed autologous cytotoxicity. Leuk Res 19:73, 1995.
Kaur, I., Voss, S.D., Gupta, R.S., Schell, K., Fisch, P. and Sondel, P.M. Human peripheral γδ T cells recognize hsp60 molecules on Daudi Burkitt's lymphoma cells. J. Immunol. 150:2046,1993.
Kitayama, J., Atomi, Y., Nagawa, H., Kuroda, A., Mutoh, T., Minami, M., Juji, T. Functional analysis of TcRγδ+ T cells in tumour-infiltrating lymphocytes (TIL) of human pancreatic cancer. Clin Exp Immunol 93:442, 1993.
Lamb, L.S., Henslee-Downey, P.J., Parrish, R.S., Godder, K., Thompson, J., Lee, C., Gee, A.P. Increased frequency of TCR gamma delta + T cells in disease-free survivors following T cell-depleted, partially mismatched, related donor bone marrow transplantation for leukemia. J Hematother 5:503, 1996.
Lang, F., Peyrat, M.A., Constant, P., Davodeau, F., David-Ameline, J., Poquet, Y., Vie, H., Fournie, J.J., Bonneville, M. J Immunol 154:5986,1995.
Orsini, D.L.M., Res, P.C.M., Van Laar, J.M., Muller, L.M., Soprano, A.E.L., Kooy, Y.M.C., Tak, P.P. and Koning, F. A subset of Vδ1+ T cells proliferates in response to Epstein-Barr virus-transformed B cell lines in vitro. Scand. J. Immunol. 38:335,1993.
Penninger, J., Wen, T., Timms, E., Potter, J., Wallace, V.A., Matsuyama, T., Ferrick, D., Sydora, B., Kronenberg, M. Mak, T.W. Spontaneous resistance to acute T-cell leukaemias in TCRδ1.1Jγ4Cγ4 transgenic mice. Nature 375:241, 1995.
Salerno, A. and Dieli, F. Role of γδ T lymphocytes in immune response in humans and mice. Crit. Rev. Immunol. 18:327, 1998.
Suzuki, Y., Fujimiya, Y., Ohno, T., Katakura, R. and Yoshimoto, T. Enhancing effect of tumor necrosis factor (TNF)-α, but not IFN-γ, on the tumor-specific cytotoxicity of γδT cells from glioblastoma patients. Cancer Lett. 140:161, 1999.
Wallace, M., Malkovsky, M., Carding, S.R. Gamma/delta T lymphocytes in viral infections. J. Leuk Biol 58:277, 1995.
Yamaguchi, T., Fujimiya, Y., Suzuki, Y., Katakura, R. and Ebina, T. A simple method for the propagation and purification of γδT cells from the peripheral blood of glioblastoma patients using solid-phase anti-CD3 antibody and soluble IL-2. J. Immunol. Meth. 205:19,1997.
Yamaguchi, T., Suzuki, Y., Katakura, R., Ebina, T., Yokoyama, J. and Fujimiya, Y. Interleukin-15 effectively potentiates the in vitro tumor-specific activity and proliferation of peripheral blood γδT cells isolated from glioblastoma patients. Cancer Immunol. Immunother. 47:97, 1998.
Yu,S., He, W., Chen, J., Zhang, F. and Ba, D. Expansion and immunological study of human tumor infiltrating gamma/delta T lymphocytes in vitro. Int. Arch. Allergy Immunol. 119:31,1999.
Zocchi, M.R., Ferrarini, M., Rugarli, C. Selective lysis of the autologous tumor by δTCS1 + γδ + tumor-infiltrating lymphocytes from human lung carcinomas. Eur J Immunol 20:2685, 1990.

## Claims

1. A method for expanding TcRγδ⁺ T cells in a starting sample comprising:
(1) culturing cells in the starting sample in a first culture medium comprising a T cell mitogen, interleukin-2 and interleukin-7; and
(2) culturing the cells obtained in step (1) in a second culture medium comprising interleukin-2 and interleukin-7 to expand TcRγδ⁺ T cells.

2. A method according to claim 1 further comprising enriching the cells in the starting sample for T cells prior to step (1).

3. A method according to any one of claims 1 to 2 further comprising enriching the cells in the starting sample for TcRγδ⁺ T cells prior to step (1).

4. A method according to any one of claims 1 to 3 further comprising depleting the cells in the starting sample of CD14⁺, CD16⁺, CD19⁺, CD33⁺, CD56⁺ and glycophorin A⁺ cells prior to step (1).

5. A method according to any one of claims 1 to 4 further comprising depleting the cells in the starting sample of TcRαβ⁺ T cells prior to step (1).

6. A method according to any one of claims 1 to 5 further comprising depleting the cells in the starting sample of non-TcRγδ⁺ T cells prior to step (1).

7. A method according to any one of claims 1 to 6 wherein the starting sample is blood or tissue or fractions thereof.

8. A method according to claim 7 wherein the starting sample is selected from peripheral blood, umbilical cord blood, bone marrow, lymphoid tissue, epithelia, thymus, liver, spleen, cancerous tissue, infected tissue, lymph node tissue or fractions thereof.

9. A method according to claim 7 or 8 wherein the starting sample is human peripheral blood or a fraction thereof.

10. A method according to any one of claims 1 to 9 wherein the starting sample is low density mononuclear cells.

11. A method according to any one of claims 1 to 10 wherein in the first culture medium the T cell mitogen is present in an amount from about 0.01 to about 100 µg/ml and wherein in the first and second culture media the interleukin-2 is present in an amount from about 0.1 to about 1000 ng/ml and the interleukin-7 is present in an amount from about 0.1 to about 1000 ng/ ml.

12. A method according to any one of claims 1 to 10 wherein in the first culture medium the T cell mitogen is present in an amount from about 0.1 to about 50 µg/ml and wherein in the first and second culture media the interleukin-2 is present in an amount from about 1 to about 100 ng/ml and the interleukin-7 is present in an amount from about 1 to about 100 ng/ml.

13. A method according to any one of claims 1 to 10 wherein in the first culture medium the T cell mitogen is present in an amount from about 0.5 to about 10 µg/ml and wherein in the first and second culture media the interleukin-2 is present in an amount from about 2 to about 50 ng/ml and the interleukin-7 is present in an amount from about 2 to about 50 ng/ml.

14. A method according to any one of claims 1 to 13 wherein the first culture medium comprises 1 µg/mL of a T cell mitogen and wherein in the first and second culture media 10 ng/mL of interleukin-2 and 10 ng/mL of interleukin-7.

15. A method according to any one of claims 1 to 14 wherein said first and second culture media further comprises an additional growth factor.

16. A method according to claim 15 wherein said additional growth factor is IL-4 or IL-15 or a mimetic or functional equivalent thereof.

17. A method according to any one of claims 1 to 14 wherein said first and second culture media further comprises two additional growth factors.

18. A method according to claim 17 wherein said two additional growth factors are IL-4 and IL-15, or a mimetic or functional equivalent thereof.

19. A method according to any one of claims 1 to 18 wherein the T cell mitogen is a plant lectin.

20. A method according to claim 19 wherein the plant lectin is concanavalin A.

21. A method according to any one of claims 1 to 18 wherein the T cell mitogen is an antibody or a fragment thereof.

22. A method according to claim 21 wherein the antibody binds to CD3 or a fragment thereof.

23. A method according to any one of claims 1 to 22 wherein the first and second culture media further contains serum or plasma.

24. A method according to claim 23 wherein the serum or plasma is present in an amount from about 1 to about 25% by volume.

25. A method according to claim 23 wherein the serum or plasma is present in an amount from about 2 to about 20% by volume.

26. A method according to claim 23 wherein the serum or plasma is present in an amount from about 2.5 to about 10% by volume.

27. A method according to claim 23 wherein the serum or plasma is present in an amount of about 5% by volume.

## Patentansprüche

1. Verfahren zum Expandieren von TcRγδ⁺-T-Zellen in einer Ausgangsprobe, welches folgendes umfasst:
(1) Kultivieren von Zellen in der Ausgangsprobe in einem ersten Kulturmedium, welches ein T-Zell-Mitogen, Interleukin-2 und Interleukin-7 umfasst, und
(2) Kultivieren der in Stufe (1) erhaltenen Zellen in einem zweiten Kulturmedium, welches Interleukin-2 und Interleukin-7 umfasst, um TcRγδ⁺-T-Zellen zu expandieren.

2. Verfahren nach Anspruch 1, welches vor Stufe (1) weiterhin umfasst, dass man die Zellen in der Ausgangsprobe hinsichtlich T-Zellen anreichert.

3. Verfahren nach einem der Ansprüche 1 bis 2, welches vor Stufe (1) weiterhin umfasst, dass man die Zellen in der Ausgangsprobe hinsichtlich TcRγδ⁺-T-Zellen anreichert.

4. Verfahren nach einem der Ansprüche 1 bis 3, welches vor Stufe (1) weiterhin umfasst, dass man die Zellen in der Ausgangsprobe hinsichtlich CD14⁺-, CD16⁺-, CD19⁺-, CD33⁺-, CD56⁺- und Glycophorin-A⁺-Zellen abreichert.

5. Verfahren nach einem der Ansprüche 1 bis 4, welches vor Stufe (1) weiterhin umfasst, dass man die Zellen in der Ausgangsprobe hinsichtlich TcRαβ⁺-T-Zellen abreichert.

6. Verfahren nach einem der Ansprüche 1 bis 5, welches vor Stufe (1) weiterhin umfasst, dass man die Zellen in der Ausgangsprobe hinsichtlich Nicht-TcRγδ⁺-T-Zellen abreichert.

7. Verfahren nach einem der Ansprüche 1 bis 6, wobei die Ausgangsprobe Blut oder Gewebe oder Fraktionen davon ist.

8. Verfahren nach Anspruch 7, wobei die Ausgangsprobe unter Peripherblut, Nabelschnurblut, Knochenmark, Lymphoidgewebe, Epithelien, Thymus, Leber, Milz, kanzerösem Gewebe, infiziertem Gewebe, Lymphknotengewebe oder Fraktionen davon ausgewählt ist.

9. Verfahren nach Anspruch 7 oder 8, wobei die Ausgangsprobe humanes Peripherblut oder eine Fraktion davon ist.

10. Verfahren nach einem der Ansprüche 1 bis 9, wobei die Ausgangsprobe mononukleäre Zellen geringer Dichte ist.

11. Verfahren nach einem der Ansprüche 1 bis 10, wobei in dem ersten Kulturmedium das T-Zell-Mitogen in einer Menge von etwa 0,01 bis etwa 100 µg/ml vorliegt und wobei in dem ersten und dem zweiten Kulturmedium das Interleukin-2 in einer Menge von etwa 0,1 bis etwa 1000 ng/ml vorliegt und das Interleukin-7 in einer Menge von etwa 0,1 bis etwa 1000 ng/ml vorliegt.

12. Verfahren nach einem der Ansprüche 1 bis 10, wobei in dem ersten Kulturmedium das T-Zell-Mitogen in einer Menge von etwa 0,1 bis etwa 50 µg/ml vorliegt und wobei in dem ersten und dem zweiten Kulturmedium das Interleukin-2 in einer Menge von etwa 1 bis etwa 100 ng/ml vorliegt und das Interleukin-7 in einer Menge von etwa 1 bis etwa 100 ng/ml vorliegt.

13. Verfahren nach einem der Ansprüche 1 bis 10, wobei in dem ersten Kulturmedium das T-Zell-Mitogen in einer Menge von etwa 0,5 bis etwa 10 µg/ml vorliegt und wobei in dem ersten und dem zweiten Kulturmedium das Interleukin-2 in einer Menge von etwa 2 bis etwa 50 ng/ml vorliegt und das Interleukin-7 in einer Menge von etwa 2 bis etwa 50 ng/ml vorliegt.

14. Verfahren nach einem der Ansprüche 1 bis 13, wobei das erste Kulturmedium 1 µg/ml eines T-Zell-Mitogens umfasst und wobei in dem ersten und dem zweiten Kulturmedium 10 ng/ml Interleukin-2 und 10 ng/ml Interleukin-7 vorliegen.

15. Verfahren nach einem der Ansprüche 1 bis 14, wobei das erste und das zweite Kulturmedium weiterhin einen zusätzlichen Wachstumsfaktor umfassen.

16. Verfahren nach Anspruch 15, wobei der zusätzliche Wachstumsfaktor IL-4 oder IL-15 oder ein Mimetikum oder funktionelles Äquivalent davon ist.

17. Verfahren nach einem der Ansprüche 1 bis 14, wobei das erste und das zweite Kulturmedium weiterhin zwei zusätzliche Wachstumsfaktoren umfassen.

18. Verfahren nach Anspruch 17, wobei die zwei zusätzlichen Wachstumsfaktoren IL-4 und IL-15 oder ein Mimetikum oder ein funktionelles Äquivalent davon sind.

19. Verfahren nach einem der Ansprüche 1 bis 18, wobei das T-Zell-Mitogen ein Pflanzenlectin ist.

20. Verfahren nach Anspruch 19, wobei das Pflanzenlectin Concanavalin A ist.

21. Verfahren nach einem der Ansprüche 1 bis 18, wobei das T-Zell-Mitogen ein Antikörper oder ein Fragment davon ist.

22. Verfahren nach Anspruch 21, wobei der Antikörper an CD3 oder ein Fragment davon bindet.

23. Verfahren nach einem der Ansprüche 1 bis 22, wobei das erste und das zweite Kulturmedium weiterhin Serum oder Plasma enthalten.

24. Verfahren nach Anspruch 23, wobei das Serum oder Plasma in einer Menge von etwa 1 bis etwa 25 Volumen-% vorliegt.

25. Verfahren nach Anspruch 23, wobei das Serum oder Plasma in einer Menge von etwa 2 bis etwa 20 Volumen-% vorliegt.

26. Verfahren nach Anspruch 23, wobei das Serum oder Plasma in einer Menge von etwa 2,5 bis etwa 10 Volumen-% vorliegt.

27. Verfahren nach Anspruch 23, wobei das Serum oder Plasma in einer Menge von etwa 5 Volumen-% vorliegt.

## Revendications

1. Procédé pour réaliser une expansion de cellules T TcRγδ⁺ dans un échantillon de départ, comprenant les étapes suivantes :
(1) cultiver des cellules dans l'échantillon de départ dans un premier milieu de culture comprenant un mitogène de cellules T, l'interleukine-2 et l'interleukine-7 ; et
(2) cultiver les cellules obtenues à l'étape (1) dans un second milieu de culture comprenant l'interleukine-2 et l'interleukine-7 afin de réaliser l'expansion des cellules T TcRγδ⁺.

2. Procédé selon la revendication 1, comprenant en outre d'enrichir les cellules dans l'échantillon de départ en cellules T avant l'étape (1).

3. Procédé selon l'une quelconque des revendications 1 à 2, comprenant en outre d'enrichir les cellules dans l'échantillon de départ en cellules T TcRγδ⁺ avant l'étape (1).

4. Procédé selon l'une quelconque des revendications 1 à 3, comprenant en outre de dépléter les cellules dans l'échantillon de départ de cellules CD14⁺, CD16⁺, CD19⁺, CD33⁺, CD56⁺ et glycophorine A⁺ avant l'étape (1).

5. Procédé selon l'une quelconque des revendications 1 à 4, comprenant en outre de dépléter les cellules dans l'échantillon de départ de cellules T TcRαβ⁺ avant l'étape (1).

6. Procédé selon l'une quelconque des revendications 1 à 5, comprenant en outre de dépléter les cellules dans l'échantillon de départ de cellules T non-TcRγδ⁺ avant l'étape (1).

7. Procédé selon l'une quelconque des revendications 1 à 6, où l'échantillon de départ est du sang ou du tissu ou des fractions de ceux-ci.

8. Procédé selon la revendication 7, où l'échantillon de départ est sélectionné à partir de sang périphérique, de sang du cordon ombilical, de moelle osseuse, de tissu lymphoïde, d'épithélium, de thymus, de foie, de rate, de tissu cancéreux, de tissu infecté, de tissu de ganglion lymphatique ou de fractions de ceux-ci.

9. Procédé selon la revendication 7 ou 8, où l'échantillon de départ est du sang périphérique humain ou une fraction de celui-ci.

10. Procédé selon l'une quelconque des revendications 1 à 9, où l'échantillon de départ est des cellules mononucléaires de faible densité.

11. Procédé selon l'une quelconque des revendications 1 à 10, dans lequel dans le premier milieu de culture, le mitogène de cellules T est présent en une quantité comprise entre environ 0,01 et environ 100 µg/ml et dans lequel, dans le premier et le second milieux de culture, l'interleukine-2 est présente en une quantité comprise entre environ 0,1 et environ 1000 ng/ml et l'interleukine-7 est présente en une quantité comprise entre environ 0,1 et environ 1000 ng/ml.

12. Procédé selon l'une quelconque des revendications 1 à 10, dans lequel dans le premier milieu de culture, le mitogène de cellules T est présent en une quantité comprise entre environ 0,1 et environ 50 µg/ml, et dans lequel dans le premier et le second milieux de culture, l'interleukine-2 est présente en une quantité comprise entre environ 1 et environ 100 ng/ml et l'interleukine-7 est présente en une quantité comprise entre environ 1 et environ 100 ng/ml.

13. Procédé selon l'une quelconque des revendications 1 à 10, dans lequel dans le premier milieu de culture, le mitogène de cellules T est présent en une quantité comprise entre environ 0,5 et environ 10 µg/ml, et dans lequel dans le premier et le second milieux de culture, l'interleukine-2 est présente en une quantité comprise entre environ 2 et environ 50 ng/ml et l'interleukine-7 est présente en une quantité comprise entre environ 2 et environ 50 ng/ml.

14. Procédé selon l'une quelconque des revendications 1 à 13, dans lequel le premier milieu de culture comprend 1 µg/ml d'un mitogène de cellules T et dans lequel dans le premier et le second milieux de culture, 10 ng/ml d'interleukine-2 et 10 ng/ml d' interleukine-7.

15. Procédé selon l'une quelconque des revendications 1 à 14, dans lequel lesdits premier et second milieux de culture comprennent en outre un facteur de croissance supplémentaire.

16. Procédé selon la revendication 15, dans lequel ledit facteur de croissance supplémentaire est l'IL-4 ou l'IL-15 ou un mimétique ou équivalent fonctionnel de celles-ci.

17. Procédé selon l'une quelconque des revendications 1 à 14, dans lequel lesdits premier et second milieux de culture comprennent en outre deux facteurs de croissance supplémentaires.

18. Procédé selon la revendication 17, dans lequel lesdits deux facteurs de croissance supplémentaires sont l'IL-4 et l'IL-15, ou un mimétique ou équivalent fonctionnel de celles-ci.

19. Procédé selon l'une quelconque des revendications 1 à 18, dans lequel le mitogène de cellules T est une lectine végétale.

20. Procédé selon la revendication 19, dans lequel la lectine végétale est la concanavaline A.

21. Procédé selon l'une quelconque des revendications 1 à 18, dans lequel le mitogène de cellules T est un anticorps ou un fragment de celui-ci.

22. Procédé selon la revendication 21, dans lequel l'anticorps se lie à CD3 ou à un fragment de celui-ci.

23. Procédé selon l'une quelconque des revendications 1 à 22, dans lequel le premier et le second milieux de culture contiennent en outre du sérum ou du plasma.

24. Procédé selon la revendication 23, dans lequel le sérum ou le plasma est présent en une quantité comprise entre environ 1 et environ 25 % en volume.

25. Procédé selon la revendication 23, dans lequel le sérum ou le plasma est présent en une quantité comprise entre environ 2 et environ 20 % en volume.

26. Procédé selon la revendication 23, dans lequel le sérum ou le plasma est présent en une quantité comprise entre environ 2,5 et environ 10 % en volume.

27. Procédé selon la revendication 23, dans lequel le sérum ou le plasma est présent en une quantité d'environ 5 % en volume.
